# EUROPÄISCHE PATENTSCHRIFT

(11) **EP 1 143 825 B1**
(45) Veröffentlichungstag und Bekanntmachung des Hinweises auf die Patenterteilung: **13.04.2005**
(21) Anmeldenummer: 00943569.4
(22) Anmeldetag: 08.05.2000
(51) Int. Cl.: A45D 31/00, A45D 29/00

(54) **LUMINESZIERENDER FINGERNAGEL**
LUMINESCENT FINGERNAIL
ONGLE LUMINESCENT

(30) Priorität: 19.11.1999 DE 19955626
(43) Veröffentlichungstag der Anmeldung: 17.10.2001
(73) Patentinhaber: Seidenbusch, Richard, 92237 Sulzbach-Rosenberg (DE)
(72) Erfinder: Seidenbusch, Richard, 92237 Sulzbach-Rosenberg (DE)
(74) Vertreter: Böhme, Volker, Dipl.-Ing.
(86) Internationale Anmeldenummer: PCT/DE2000/001439
(87) Internationale Veröffentlichungsnummer: WO 2001/037698

(56) Entgegenhaltungen:
- WO-A-99/21453
- GB-A- 1 280 700
- PATENT ABSTRACTS OF JAPAN vol. 011, no. 246 (C-439), 11. August 1987 (1987-08-11) & JP 62 053916 A (SHIGERU SUGIMOTO;OTHERS: 01), 9. März 1987 (1987-03-09)
- DATABASE EPODOC [Online] EUROPEAN PATENT OFFICE, THE HAGUE, NL; CN1161830 A, 15. Oktober 1997 (1997-10-15) HE JIGPING: XP002150997
- PATENT ABSTRACTS OF JAPAN vol. 1998, no. 02, 30. Januar 1998 (1998-01-30) & JP 09 266815 A (MOTODA KAZUE), 14. Oktober 1997 (1997-10-14)
- DATABASE WPI Section Ch, Week 199749 Derwent Publications Ltd., London, GB; Class A60, AN 1997-527314 XP002150998 & CN 1 126 746 A (XIAO Z), 17. Juli 1996 (1996-07-17)
- PATENT ABSTRACTS OF JAPAN vol. 1999, no. 02, 26. Februar 1999 (1999-02-26) & JP 10 292257 A (TORAY IND INC), 4. November 1998 (1998-11-04)

## Beschreibung

Die Erfindung betrifft einen Fingernagel, bei dem ein Nagelgrundkörper mit einem nachleuchtenden Lumineszenz-Pigment in einem Trägermaterial versehen ist.

Gemäß JP-A-10 087 437 wird auf einen Nagel ein Nagellack aufgebracht, der mindestens ein nachleuchtendes Lumineszenz-Pigment und ein färbendes perlartiges Pigment aufweist. Dieser aufgebrachte Nagellack ist letztendlich einfarbig und insofern für einen natürlichen Fingernagel gut geeignet, bei dem eine Person den zunächst flüssigen Nagellack auf den natürlichen Nagelgrundkörper manuell aufbringt. Es ist nun aber auch erwünscht, wenn ein Fingernagel mit zwei oder mehr verschiedenen Farben verziert ist, die vermischt miteinander oder auch nebeneinander, d.h. gesondert voneinander vorgesehen sind.

Eine Aufgabe der Erfindung ist es daher, einen Fingernagel der eingangs genannten Art zu schaffen, der mit mindestens zwei verschiedenen lumineszierenden Farben versehen ist. Der erfindungsgemäße Fingernagel ist, diese Aufgabe lösend, dadurch gekennzeichnet, daß ein künstlicher Kunststoff-Nagelgrundkörper mit der Summe eines stark nachleuchtenden (≥ 40 mcd/m2 nach 60 min) farbigen Lumineszenz-Pigment auf Erdalkalialuminat-Basis und eines weiteren andersfarbigen Lumineszenz-Pigments versehen ist.

Bei dem erfindungsgemäßen, z.B. aufklebbaren, künstlichen Fingernagel sind zwei oder mehr lumineszierende Farben vorgesehen. Dadurch wird die Zierwirkung des Fingernagels erheblich gesteigert. Die verschiedenen Farben sind z.B. seitlich nebeneinander oder übereinander angeordnet oder gemischt miteinander vorgesehen. Das Aufbringen von zwei oder mehr verschiedenen Farben auf einen natürlichen Nagelgrundkörper läßt sich von einer Person manuell nur unter großen Schwierigkeiten durchführen. Deshalb sind bei der Erfindung die verschiedenen Farben an einem künstlichen Kunststoff-Nagelgrundkörper vorgesehen, der maschinell gefertigt wird, wobei der künstliche Fingernagel maschinell mit den verschiedenen Farben versehen wird. Die zwei oder mehr verschiedenen lumineszierenden Farben sind z.B. nebeneinander, d.h. gesondert voneinander oder eigenständig bzw. nicht gemischt vorgesehen.

Bei einer ersten Ausführungsform der Erfindung ist der künstliche Nagelgrundkörper mit einem aufgebrachten Nagellack versehen. Der starklumineszierende aufgebrachte Nagellack ist definiert durch eingebrachte stark nachleuchtende Pigmente (≥ 40 mcd/m2 nach 60 min) in wasserfreien Ein- oder Zweikomponenten-Nagellack, um einen deutlichen Nachleuchteffekt der Nägel zu erzielen.

Farblose oder transparentfarbene und wasserfreie Nagellacke werden mit stark nachleuchtenden Pigmenten (≥ 40 mcd/m2 nach 60 min, z.B. Marke Lumilux SN-F2) versetzt. Je nach Qualität und Quantität der zugeführten Pigmente läßt sich die Leuchtdichte steuern. Ein additiver Zusatz von weiteren Pigmenten geringerer Leuchtdichte (z.B. Effekt Blau N, Sipi Gelb SN, Effekt Rot N 100) kann gelbe, blaue oder rote Nachleuchteffekte erzielen. Gerade bei der Teenager- und Disco-Tekkno-Generation kann ein solcher intensiv und lang nachleuchtender Nagellack zu einem überraschenden Run führen.

Der starklumineszierende Kunststoff-Fingernagel ist so ausgebildet, daß die Leuchtpigmente oder Pigmentmischungen in aufklebbare Kunststoff-Fingernägel eingebracht oder alternativ als Nachleuchtlacke oder Nachleuchtkunststoffe auf die Nagelgrundkörper aufgebracht sind. Die überwiegend sehr hitzebeständigen Pigmente (≤ 500°C) sind z.B. in Kunststoffe eingemischt, aus denen die stark nachleuchtenden Kunststoff-Fingernägel hergestellt sind.

Bei einer Ausführungsform der Erfindung ist vorgesehen eine ausschließliche Applikation oder Implikation von lumineszierenden Pigmentkombinationen (2 - 5 lumineszierende Komponenten) aus starklumineszierenden Pigmenten der Erdalkalialuminatgruppe und schwachlumineszierende Pigmente aus Metall-Sulfid-Abkömmlingen auf oder in Kunststoff-Fingernägel. Z.B. übertreffen die Pigmentkombinationen (Pigmentabmischungen oder Pigmentschichtenüberlagerungen) prozentual mit 51 - 85 % wt (> 50 % wt) das Trägergewicht. Der Träger soll keine (0 %) zusätzlichen nichtlumineszierenden Farbpigmente enthalten.

Eine starklumineszierende Mehrkomponenten-Ausführungsform wird ausschließlich mit Pigmentabmischungen aus stark nachleuchtenden (SN) Pigmenten aus Erdalkalialuminaten und schwachlumineszierenden (N) Pigmenten (aus Metall-Sulfid-Komponenten) komponiert. Je nach Qualität und Quantität der Pigmentabmischung (mehrere 100 Kombinationen möglich: z.B. 80 % SN Grün + 15 % N Rot + 5 % N Gelb; z.B. 70 % SN Blau + 15 % N Blau + 10 % N Rot + 5 % N Gelb) lassen sich Leuchtdichte und Leuchtfarbe für gelbe, blaue, rot-orange, violette, grüne Nachleuchtfarbtöne steuern. Aus drei Tageslichtpastellgrundtönungen (grün - beige; ocker - rosa; gelblich) lassen sich mehrere Dutzend Nachleuchtfarben produzieren. Als alternative wie auch additive Kombinationsmethode zur Pigmentabmischung ist die Überlagerung von 2 - 4 verschiedenen lumineszierenden Pigmentschichten zur Farbsteuerung möglich: z.B. SN-Unterlegschicht - feine N-Auflageschicht - oder N-SN-N oder SN-N-SN. Diese Überlagerungsvariante ist vorzugsweise bei der Kunstnägelherstellung in Betracht zu ziehen.

Als neuheitlich anzusehen ist die ausschließliche Verwendung von lumineszierenden Farbpigmenten, die ohne zusätzliche nichtlumineszierende Farbträger realisierbar ist und die zu dem ausschließlich in Kombination (Pigmentabmischungen oder -überlagerungen) von mehreren (2 - 4) stark- und schwachlumineszierenden Pigmenten, mit z.B. ≥ 51 % wt, erfolgt. Diese drei Faktoren ermöglichen zum einen einen effektiven Nachleuchteffekt und zum anderen die Realisierung einer breiten Nachleuchtfarbpalette mit mehreren Dutzend Nachleuchtfarbtönungen und Leuchtstrukturierungen.

Der Kunststoff der Beschichtung des künstlichen Fingernagels ist z.B. Silikon. Während bei einem Kunststoff des Fingernagels der Lumineszenz-Pigmentanteil auch kleiner als 50 % sein kann, ist der Lumineszenz-Pigmentanteil bei einem Nagellack vorzugsweise größer als 50 %

## Patentansprüche

1. Fingernagel,
der einen Nagelgrundkörper aus Kunststoff umfasst,
wobei der Nagelgrundkörper wenigstens zwei verschiedene lumineszierende Farben aufweist und
wobei wenigstens eine lumineszierende Farbe ein stark nachleuchtendes, farbiges Lumineszenz-Pigment mit einer Leuchtdichte ≥ 40 mcd/m² nach 60 min ist und wenigstens eine weitere lumineszierende Farbe ein stark nachleuchtendes, farbiges Lumineszenz-Pigment oder ein schwach nachleuchtendes, farbiges Lumineszenz-Pigment ist.

2. Fingernagel nach Anspruch 1,
wobei das wenigstens eine stark nachleuchtende, farbige Lumineszenz-Pigment auf einem Erdalkalialuminat basiert.

3. Fingernagel nach Anspruch 1 oder Anspruch 2,
wobei die lumineszierenden Farben seitlich nebeneinander angeordnet sind und/oder übereinander angeordnet sind und/oder gemischt miteinander vorgesehen sind.

4. Fingernagel nach einem oder mehren der vorhergehenden Ansprüche,
wobei die Lumineszenz-Pigmente in den Nagelgrundkörper eingebracht sind oder in einem Trägermaterial auf den Nagelgrundkörper aufgebracht sind, insbesondere als Nachleuchtlacke oder Nachleuchtkunststoffe.

5. Fingernagel nach einem oder mehren der vorhergehenden Ansprüche,
wobei wenigstens ein schwach nachleuchtendes, farbiges Lumineszenz-Pigment auf einem Metall-Sulfid basiert.

6. Fingernagel nach einem oder mehren der vorhergehenden Ansprüche,
wobei aus drei Pastellgrundtönungen bei Tageslicht, insbesondere grün-beige, ocker-rosa oder gelblich, mehrere Dutzend Nachleuchtfarben bei Dunkelheit produziert werden.

7. Fingernagel nach einem oder mehren der vorhergehenden Ansprüche,
wobei wenigstens zwei verschiedene lumineszierende Pigmentschichten vorgesehen sind.

8. Fingernagel nach Anspruch 7,
wobei eine erste stark nachleuchtende (SN) und eine zweite schwach nachleuchtende (N) oder eine erste stark nachleuchtende (SN), eine zweite schwach nachleuchtende (N) und eine dritte starknachleuchtende (SN) oder eine erste schwach nachleuchtende (N), eine zweite stark nachleuchtende (SN) und eine dritte schwach nachleuchtende (N) Pigmentschicht übereinander angeordnet sind, insbesondere im Nagelgrundkörper.

9. Fingernagel nach einem oder mehren der vorhergehenden Ansprüche,
wobei überwiegend stark nachleuchtende, farbige Lumineszenz-Pigmente verwendet werden, insbesondere zu 70 % bis 80 %.

10. Fingernagel nach einem oder mehren der vorhergehenden Ansprüche,
wobei der Gewichtsanteil der Lumineszenz-Pigmente im Trägermaterial größer 50% wt, insbesondere zwischen 51 und 85% wt, oder kleiner 50% wt beträgt und/oder der Gewichtsanteil der Lumineszenz-Pigmente im Nagelgrundkörper kleiner 50% wt beträgt.

11. Fingernagel nach einem oder mehren der vorhergehenden Ansprüche,
wobei in den Nagelgrundkörper und/oder in das Trägermaterial eingebrachte Lumineszenz-Pigmente eine Hitzebeständigkeit von ≤ 500 °C aufweisen.

## Claims

1. A fingernail, which comprises a nail base body of plastics material, wherein the nail base body has at least two different luminescent colours and wherein at least one luminescent colour is a strongly afterglowing coloured luminescence pigment with a luminance of ≥ 40 mcd/m² after 60 min and at least one further luminescent colour is a strongly after-glowing coloured luminescence pigment or a weakly afterglowing coloured luminescence pigment.

2. A fingernail according to Claim 1, wherein the at least one strongly after-glowing coloured luminescence pigment is based on an alkaline-earth aluminate.

3. A fingernail according to Claim 1 or Claim 2, wherein the luminescent colours are arranged laterally adjacent to one another and/or are arranged one above the other and/or are provided mixed with one another.

4. A fingernail according to one or more of the preceding Claims, wherein the luminescence pigments are introduced into the nail base body or are applied in a carrier material to the nail base body, in particular in the form of afterglow lacquers or afterglow plastics materials.

5. A fingernail according to one or more of the preceding Claims, wherein at least one weakly afterglowing coloured luminescence pigment is based on a metal sulphide.

6. A fingernail according to one or more of the preceding Claims, wherein several dozen afterglow colours in the dark are produced from three pastel base shades in daylight, in particular green-beige, ochre-pink or yellowish.

7. A fingernail according to one or more of the preceding Claims, wherein at least two different luminescent pigment layers are provided.

8. A fingernail according to Claim 7, wherein a first strongly afterglowing (SN) and a second weakly afterglowing (N) pigment layer or a first strongly afterglowing (SN), a second weakly afterglowing (N) and a third strongly afterglowing (SN) pigment layer or a first weakly afterglowing (N), a second strongly afterglowing (SN) and a third weakly afterglowing (N) pigment layer are arranged one above the other, in particular in the nail base body.

9. A fingernail according to one or more of the preceding Claims, wherein predominantly strongly afterglowing coloured luminescence pigments are used, in particular up to from 70% to 80%.

10. A fingernail according to one or more of the preceding Claims, wherein the proportion by weight of the luminescence pigments in the carrier material amounts to more than 50% by weight, and in particular to between 51 and 85% by weight, or less than 50% by weight and/or the proportion by weight of the luminescence pigments in the nail base body amounts to less than 50% by weight.

11. A fingernail according to one or more of the preceding Claims, wherein luminescence pigments introduced into the nail base body and/or into the carrier material have a heat resistance of ≤ 500°C.

## Revendications

1. Ongle de doigt qui comprend un corps de base d'ongle en matière plastique, dans lequel le corps de base d'ongle comporte au moins deux couleurs luminescentes différentes et au moins une couleur luminescente est un pigment de luminescence coloré, fortement phosphorescent avec une luminance ≥ 40 mcd/m² après 60 minutes et au moins une autre couleur luminescente est un pigment de luminescence coloré, fortement phosphorescent ou un pigment de luminescence coloré, faiblement phosphorescent.

2. Ongle de doigt selon la revendication 1, dans lequel le au moins un pigment de luminescence coloré, fortement phosphorescent est à base d'un aluminate alcalino-terreux.

3. Ongle de doigt selon la revendication 1 ou la revendication 2, dans lequel les couleurs luminescentes sont placées latéralement l'une à côté de l'autre et/ou sont placées l'une sur l'autre et/ou sont prévues mélangées l'une avec l'autre.

4. Ongle de doigt selon une ou plusieurs des revendications précédentes, dans lequel les pigments de luminescence sont introduits dans le corps de base d'ongle ou sont appliqués dans un substrat sur le corps de base d'ongle, en particulier sous forme de laque phosphorescente ou de matières plastiques phosphorescentes.

5. Ongle de doigt selon une ou plusieurs des revendications précédentes, dans lequel au moins un pigment de luminescence coloré, faiblement phosphorescent est à base d'un sulfure métallique.

6. Ongle de doigt selon une ou plusieurs des revendications précédentes, dans lequel plusieurs douzaines de couleurs phosphorescentes sont produites dans l'obscurité à partir de trois nuances pastel de fond à la lumière du jour, en particulier vert-beige, ocre-rose ou jaunâtre.

7. Ongle de doigt selon une ou plusieurs des revendications précédentes, dans lequel il est prévu au moins deux couches de pigment de luminescence colorées.

8. Ongle de doigt selon la revendication 7, dans lequel une première couche de pigment fortement phosphorescente (SN) et une deuxième couche de pigment faiblement phosphorescente (N) ou une première couche de pigment fortement phosphorescente (SN), une deuxième couche de pigment faiblement phosphorescente (N) et une troisième couche de pigment fortement phosphorescente (SN) ou une première couche de pigment faiblement phosphorescente (N), une deuxième couche de pigment fortement phosphorescente (SN) et une troisième couche de pigment faiblement phosphorescente (N) sont placées les unes au dessus des autres, en particulier dans le corps de base d'ongle.

9. Ongle de doigt selon une ou plusieurs des revendications précédentes, dans lequel on utilise majoritairement des pigments de luminescence colorés fortement phosphorescents, en particulier entre 70% et 80%.

10. Ongle de doigt selon une ou plusieurs des revendications précédentes, dans lequel la proportion en poids des pigments de luminescence dans le substrat est supérieure à 50% en poids, est en particulier comprise entre 51% et 85% en poids, ou est inférieure à 50% en poids et/ou la proportion en poids des pigments de luminescence dans le corps de base d'ongle est inférieure à 50% en poids.

11. Ongle de doigt selon une ou plusieurs des revendications précédentes, dans lequel les pigments de luminescence sont introduits dans le corps de base d'ongle et/ou dans le substrat présentent une résistance à la chaleur de ≤ 500°C.
